# EUROPEAN PATENT APPLICATION

(11) **EP 1 029 508 A1**
(43) Date of publication of application: **23.08.2000**
(21) Application number: 99200473.9
(22) Date of filing: 19.02.1999
(51) Int. Cl.: A61B 17/02

(54) **Ocular speculum and method for separation of eyelids**

(71) Applicant: Melles, Gerrit Reinold Jacob, 3063 GC Rotterdam (NL)
(72) Inventor: Melles, Gerrit Reinold Jacob, 3063 GC Rotterdam (NL)
(74) Representative: Ottevangers, Sietse Ulbe

(57) **Abstract**

Ocular speculum, comprising separating means for separating the eyelids of an eye by engagement of the ocular side of the eyelids. The separating means comprise anchoring means for, in use, retaining said separating means within the conjunctival fornix of an eye. In a preferred embodiment the separating means are configured as a drape, while the anchoring means are configured as arcuate segments connected by a spring lever.

## Description

The invention relates to an ocular speculum, comprising separating means for separating the eyelids of an eye by engagement of the ocular side of the eyelids.

Such a speculum is generally known and is used in almost any type of ocular surgery to separate the eyelids in order to gain access to the eye, the peri-ocular or orbital structures.

In the known ocular speculum, the separating means comprise hooks for engagement of the ocular side of the respective upper and lower eyelid, which hooks are connected by a spring lever. In use, the hooks are placed around the eyelids and the spring lever pulls the hooks and the eyelids apart.

The known ocular speculum has a number of disadvantages that are frequently encountered during surgery. Firstly, since the ocular speculum is retained by the eyelids, looseness or movement of the eyelid can cause the separating means to be disengaged, such that the speculum is no longer effective.

Secondly, since in surgery the ocular entrance wound is commonly made at the corneal limbus or in the sclera in the area that is normally covered by the upper eyelid, the hooks of the ocular speculum engaging the upper eyelid are located in the working area. The presence of the hooks and the spring lever attached to the hooks therefore often interferes with handling of various instruments during surgery.

Thirdly, because of the anatomy of the peri-ocular structures, in particular the eyelid anatomy relatively to the eyeball, the hooks of the speculum exert an inward force onto the eyeball. This force can raise the intra-ocular pressure that in turn may lead to shifting of the intra-ocular contents toward the wound when the eye is opened, thereby increasing the risk of intra-operative complications.

Fourthly, in most ocular surgical procedures, the ocular speculum is used in combination with a plastic drape to cover the eyelashes, the eyelids and peri-ocular skin in order to obtain a sterile surgical field. Conventionally, the drape is glued onto the peri-ocular skin and the drape is incised where it covers the eye in order to gain access to the eye. Subsequently, both lips of the incised portion of the drape are tucked in between the eyeball and the ocular side of the eyelids. The ocular speculum is then placed over the drape, such that the eyelashes and the eyelids are covered by the drape and the eyelids are separated by engagement of the ocular side of the eyelids by the hooks of the speculum. Due to the anatomy of the face, the force exerted onto the drape and the eyelids during surgery and the tendency of the drape to become wrinkled, the eyelids often become at least partially exposed during surgery. This increases the risk of contamination of the surgical field, in particular when the eyelashes become exposed.

The present invention seeks to overcome the above-mentioned problems. Thereto, an ocular speculum according to the invention is characterized in that the separating means comprise anchoring means for, in use, retaining said separating means within the conjunctival fornix of an eye. By anchoring the separating means within the conjunctival fornix, i.e. the fold of the mucous membrane (the transition of the tarsal and bulbar conjunctiva) connecting the eyeball to the eyelid, stabilization of the ocular speculum can be greatly increased, greatly reducing the chance of disengagement of the ocular speculum. Also, by providing the anchoring means in the conjunctival fornix, the number of parts that extend on the outside of the eye and that hence may cause interference in the working area can be greatly reduced. Furthermore, the chance of exertion of force onto the eyeball can be greatly reduced. It should be noted that in this context the term "conjunctival fornix" is to be interpreted, where appropriate to include both the upper conjunctival fornix extending around the upper portion of the eyeball, and the lower portion of the conjunctival fornix extending around the lower portion of the eyeball, i.e. both the upper and the lower conjunctival fornices.

In an advantageous embodiment the separating means comprise a surgical drape extending from the anchoring means. This way, complete continuous coverage of the eyelids and eyelashes during surgery can relatively easily be achieved, thus greatly facilitating preservation of sterility of the surgical field. In addition, the drape can be used to cover parts of the ocular speculum extending out of the eye, such as a spring lever. This way, the chance of interference of such parts with other surgical tools can be greatly reduced.

The invention also relates to a method for separation of the eyelids of an eye and to a surgical drape.

Further features and advantageous embodiments are defined in the claims. The invention will be further elucidated on the basis of a number of preferred embodiments shown in a drawing. In the drawing:
fig. 1 is a plan view of a facial portion having an eye of which the eyelids are separated with a first embodiment of an ocular speculum according to the invention;
fig. 2 a longitudinal cross section of the facial portion according to fig. 1 along the line II-II;
fig. 3 a perspective view of the anchoring means for a second embodiment of the ocular speculum according to the invention;
fig. 4 a plan view of a surgical drape comprising the anchoring means according to fig. 3;
fig. 5 a plan view of a facial portion in which the eyelids are separated with an ocular speculum according to fig. 4;
fig. 6 a longitudinal cross section of the facial portion according to fig. 5 along the line VI-VI;
fig. 7 a third embodiment of the ocular speculum according to the invention;
fig. 7A a fourth embodiment of the ocular speculum according to the invention;
fig. 8 a fifth embodiment of the ocular speculum according to the invention; and
fig. 9 a sixth embodiment of the ocular speculum according to the invention.

The figures are schematic representations of preferred embodiments of the invention and only serve as illustrations. In the figures, identical or corresponding parts are designated by the same reference numerals.

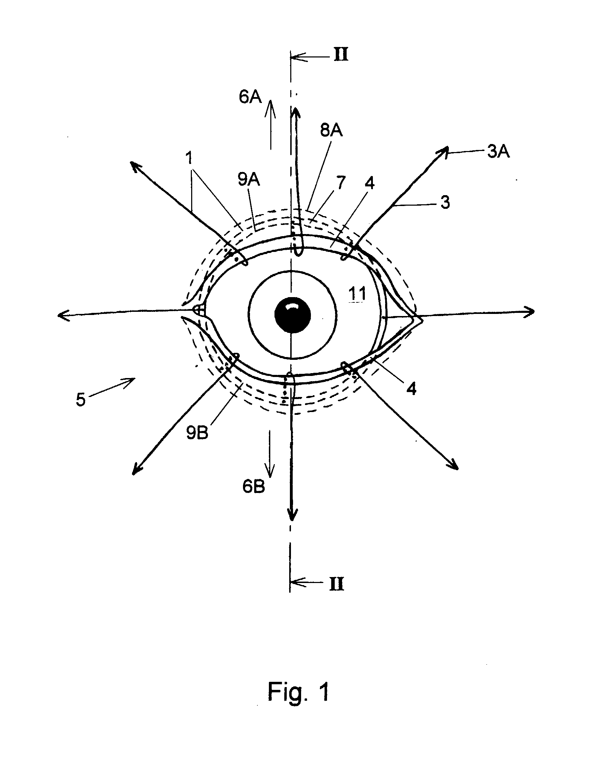

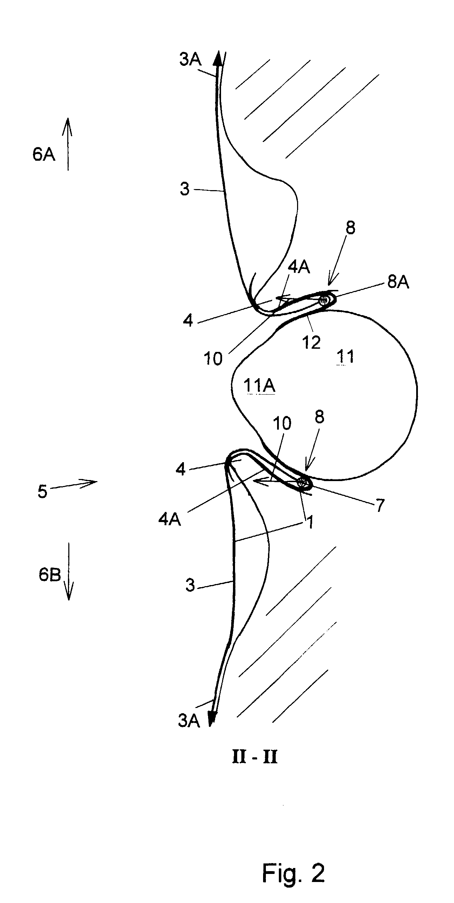

Referring to figs. 1 and 2, an ocular speculum 1 is shown. The ocular speculum 1 comprises separating means 3, in this embodiment configured as wires, for separating the eyelids 4 of an eye, globally indicated with reference numeral 5. The eyelids 4 are separated globally in the direction indicated with arrows 6A and 6B. The separating means comprise anchoring means 7 for retaining the separating means 3 within the conjunctival fornix 8 of the eye 5. In this embodiment, the anchoring means are configured as an annular ring. The inner part of the annular ring is open to give access to the anterior portion 11A of the eyeball 11. In use, the anchoring means 7 are inserted in the conjunctival fornix 8, i.e. near the fold 8A of the muscous membrane 12 connecting the eyeball 11 to the inner portion 4A of the eyelid 4. Thus, in contrast to a conventional eye lid speculum that is retained by the most anterior part of the ocular side of the eyelids, i.e. where the mucous membrane of the inner side of the eyelid merges with the facial skin, the eyelid speculum according to the invention is retained by a more posterior part of the inner eyelids, i.e. the area where the mucous membrane of the inner eyelid merges with the mucous membrane of the eyeball.

Insertion of the anchoring means 7 in the embodiment of the annular ring can be carried out by e.g. firstly inserting a top portion 9A of the ring into the conjunctival fornix 8 and, while slightly deforming the area around the conjunctival fornix 8 near the top portion 9A of the ring subsequently inserting a lower portion 9B of the annular ring into the lower portion of the conjunctival fornix 8. The separating means 3, being attached to the anchoring means 7, are now anchored in the conjunctival fornix 8. Subsequently, using the separate means, the eyelids 4 can be separated by engagement of the ocular side 4A of the eyelids 4. In this embodiment, this is carried out by pulling the wires 3 in the direction of the arrows 3A. When a flexible wire is used, the wire can e.g. be clamped, sewn or glued onto the facial skin or onto a drape, covering the face. When a relatively stiff wire 3 is used, the wire can be of such a shape that it extends radially outward previous to insertion, such that it engages the ocular side 4A of the eyelids 4 and pushes them outward during insertion. Additionally, a bendable wire can be used that, after insertion of the anchoring means 7, is bent radially outwards relatively to the anchoring means in the general direction of the arrows 3A and, optionally, is cut to the desired length.

In separated condition of the eyelids 4, the anterior portion 11A, and the peri-ocular or orbital structures are easily accessible, while the anchoring means 7 retain the separating means 3 within the conjunctival fornix 8 such that separation of the eyelids is maintained stable. In particular, the shape of the conjunctival fornix 8 and the eyelids 4 is such that the anchoring means 7 are retained in the eye 5 when a pulling force is exerted on the separating means 3. Due to the annular shape, such a pulling force results in a force in the direction of arrows 10 of the anchoring means 7, the chance of exertion of any inward pressure on the eyeball 11 being greatly reduced.

To increase the retaining capacity of the anchoring means and to reduce the chance of exertion of inward pressure on the eyeball by deformation of the anchoring means, the anchoring means are preferably made of a substantially rigid material. When an annular element is used as anchoring means, the annular element may e.g. be formed from metal or plastic. In such an embodiment the outer diameter of the annular element may e.g. vary from 18 to 30 mm and the thickness of the annular element may e.g. vary from 0.3 to 5 mm, depending on the size of the eye.

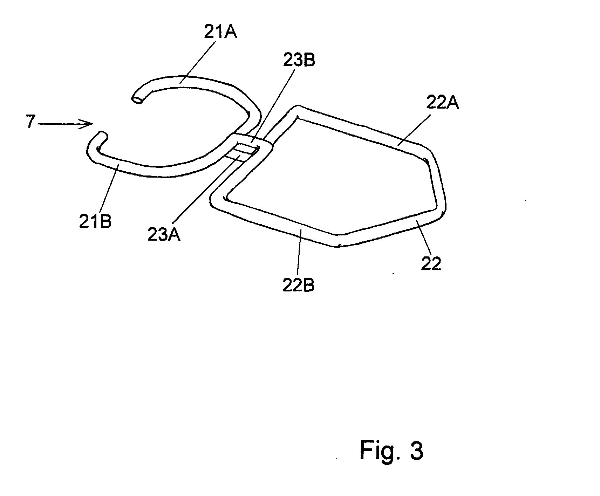

Referring to figs. 3 to 6 a second embodiment of the ocular speculum 1 is shown. The ocular speculum comprises anchoring means 7 formed as two substantially arcuate segments 21A and 21B. The segments 21A and 21B are resiliently connected by a spring lever 22. The spring lever 22 biasses the arcuate segments 21A and 21B to form a substantially arcuate structure. By pressing the legs 22A and 22B of the spring lever towards each other, the segments 21A and 21B can be moved towards each other, such that insertion of the arcuate segments 21A and 21B past the eyelids 4 into the conjunctival fornix 8 is greatly facilitated. Upon releasing the legs 22A and 22B of the spring lever 22, the segments 21A and 21B become firmly anchored in the conjunctival fornix 8 of the eye 5, thereby retaining the separating means 7, to be discussed further on, within the conjunctival fornix 8 of the eye 5. Providing the spring lever 22 with overlapping portions, such as superposed loops 23A and 23B near the corner 24 of the eye 5 i.e. near the lateral canthus of the eye, the ocular speculum 1 can be arranged such that forced closure of the eyelids 4 results in outward displacement of the arcuate segments 21A and 21B, thereby reinforcing the position of the ocular speculum 1 within the conjunctival fornix.

The arcuate segments 21A, 21B and the spring lever 22 are made of any material combining the required stiffness for retainment with sufficient biassing capacity, e.g. plastic or metal.

The arcuate segments can e.g. be formed from bent wire having a diameter of 1 mm and a length of 30 to 50 mm and a radius of curvature of e.g. 30 to 80 mm.

An advantage of an embodiment having arcuate segments that are arranged to be biassed outward is that the size of the speculum relative to the eye becomes less critical. Also, the lever can be provided with adjustment means to adjust the amount of travel of the segments and/or the spring force. Furthermore, outward biassing of the anchoring means relative to each other decreases the chance of pressure being exerted onto the eyeball by inward movement of the segments relative to each other.

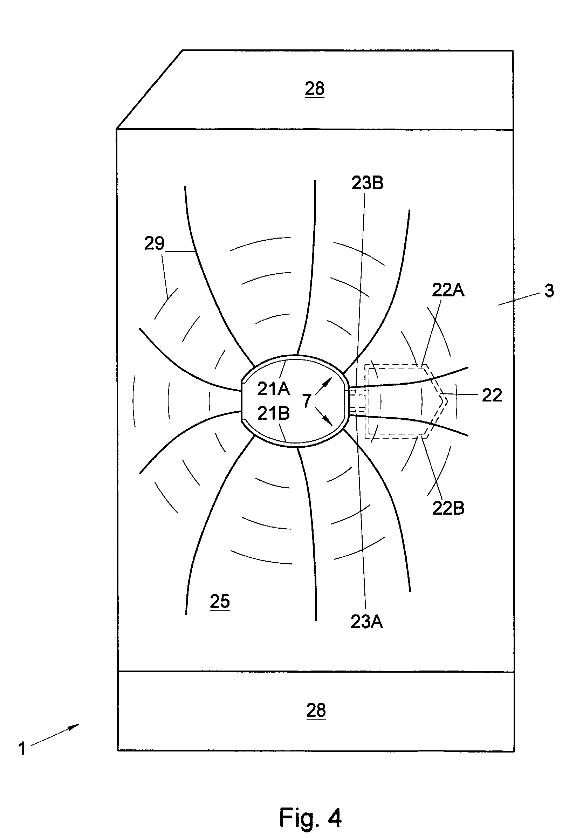

Referring to figures 4-6 it is shown that the anchoring means 7, in this embodiment configured as arcuate segments 21A and 21B, are attached to a surgical drape 25 which forms the separating means 3. In the drawing lines 29 are drawn to indicate the surface of the drape more clearly. The surgical drape 25 can be of any suitable material previously used in drapes for ocular surgery, but is preferably made of at least partially transparent plastic material. The drape 25 is attached to the anchoring means 7 in this embodiment formed as arcuate segments 21 A and 21B in such a way that an area surrounded by the anchoring means is open to accommodate the anterior portion 11A of the eyeball 11. The anchoring means 7 can be integrally formed with the drape 25, e.g. a substantially arcuate C-shaped segment or annular ring integrally moulded into the drape 25, or sewn, welded or glued thereto. In addition, the drape 25 can also be provided with pockets for removable attachment of the drape 25 to the anchoring means 7 by insertion. In the embodiment shown in fig. 4, the anchoring means 7 are formed as arcuate segments 21A and 21B made from bent wire, glued to the surgical drape 25.

By arranging the surgical drape 25 such that a facial side thereof (in fig. 4 the side opposite the plan of view) extends over the spring lever 22, in use, interference of the spring lever 22 with surgical instruments can be greatly reduced. By compression of the legs 22A and 22B of the spring lever 22, the arcuate segments 21A and 21B are moved towards each other and can be tucked under the eyelids 4 upon insertion of the anchoring means 7 into the eye. By subsequently releasing the spring lever 22 and lightly pressing the ocular speculum 1 through the passage between ocular side of the eyelids 4 and the eyeball 11, the arcuate segments 21A and 21B can relatively easily be placed in close vicinity of the fold 8A of the conjunctival fornix 8, while the portion of the drape 25 extending from the arcuate segments is automatically inserted therewith. By subsequently pulling the surgical drape 25 outwardly, the surgical drape 25 engages the ocular side 4A of the eyelids and separates the eyelids 4, while the arcuate segments 21A and 21B retain the lower portion surgical drape 25 in the conjunctival fornix 8.

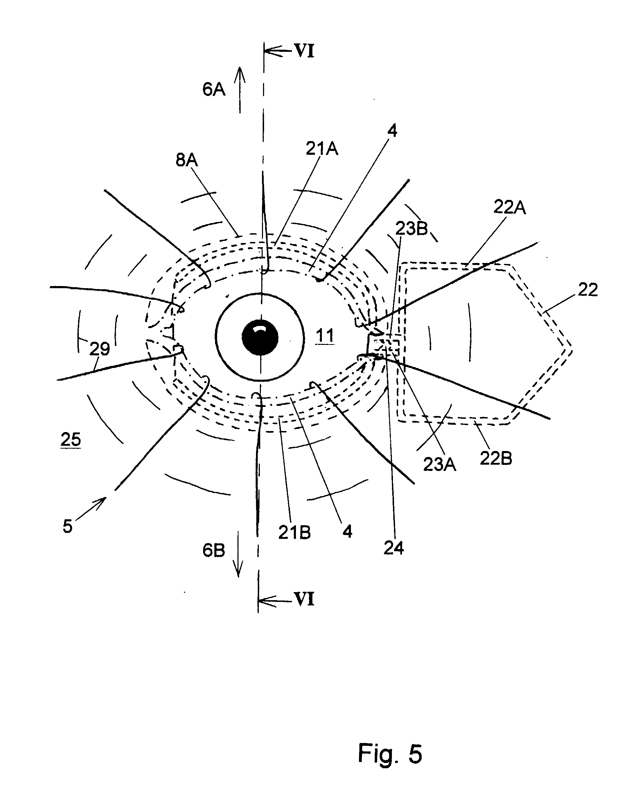

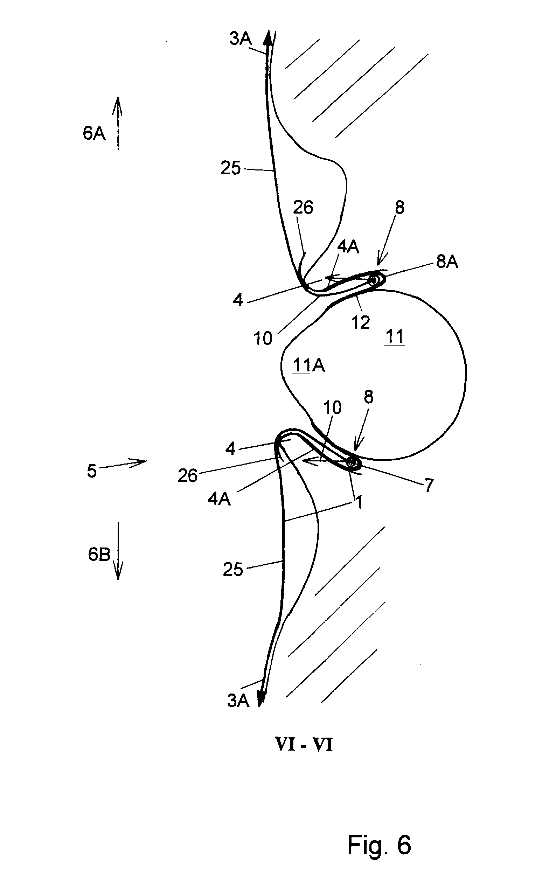

The surgical drape 25 can subsequently be attached to the facial structure. By providing the facial side of the surgical drape 25 with adhesive areas 28, attachment to the facial structure can be greatly facilitated. As indicated in figs. 5 and 6, in use, the eyelashes 26 are completely covered by the surgical drape 25 and accessibility of the eye, the peri-ocular and orbital structures is optimized. This embodiment in particular can relatively easily be integrally manufactured as a disposable product from plastic material.

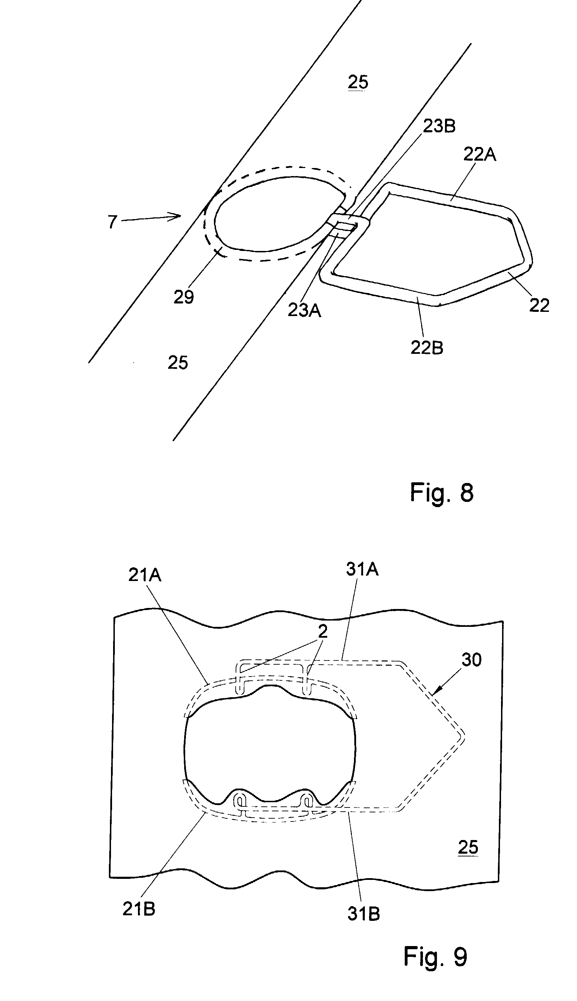

By choosing the width of the drape 25 substantially equal to the width of the eye 5 insertion of the drape can be further facilitated. In fig. 8 such a drape is shown.

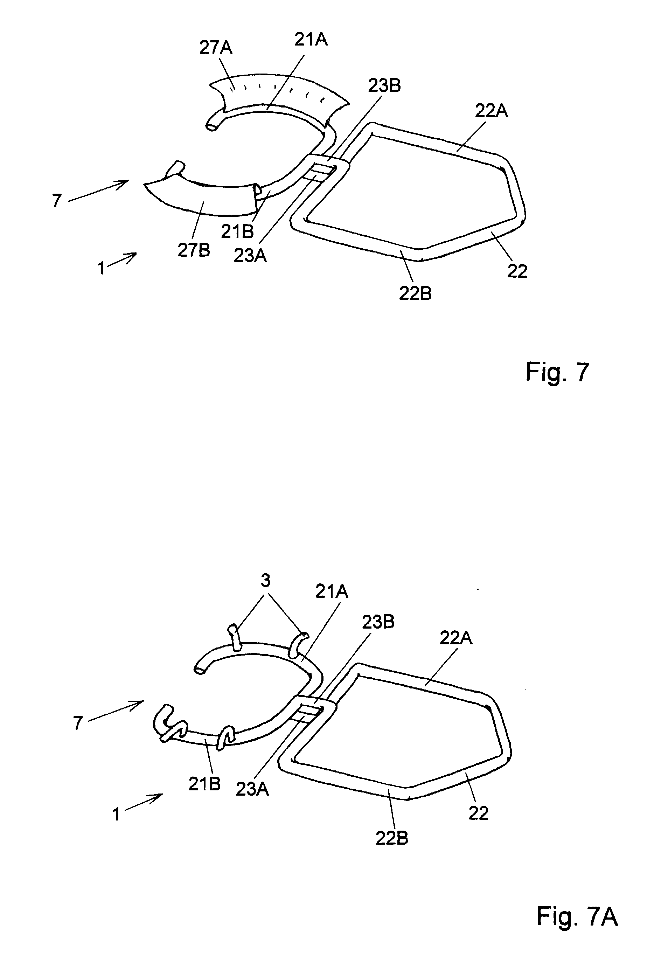

In fig. 7 a third embodiment of an ocular speculum 1 according to the invention is shown. In this embodiment the separating means 3 are configured as curved blades 27A and 27B for cooperation with the ocular portions 4A of the eyelids 4. The curved blades 27A and 27B each extend from arcuate segments 21A and 21B, forming the anchoring means 7 integrated with the separating means for insertion into the conjunctival fornix 8 of an eye 5 in a manner as described above. The arcuate segments 21A and 21B are resiliently connected by a spring lever 22 for manipulating the ocular speculum into the eye as described above. This embodiment of the ocular speculum can also be used in combination with a surgical drape 25, e.g. by coupling the surgical drape 25 to the arcuate segments 21A and 21B or to the curved blades 27A and 27B.

Fig. 7A shows a fourth embodiment of the ocular speculum according to the invention that is used the same way as the embodiment of fig. 7. However, the anchoring means carry stiff, bent wires 3 as separating means instead of the curved blades 27A and 27B.

Fig. 8 shows a fifth embodiment of the ocular speculum 1 according to the invention. Instead of arcuate segments 21A and 21B, a substantially C-shaped segment 29 is provided, i.e. an annular structure having an open or discontinuous circumference e.g. a ring having a cut in its circumference. This embodiment is also relatively easy to insert into the eye 5 and has the additional advantage of having a continuous portion extending near the corner of the eye 5 opposite to the overlapping portions 23A and 23B. Instead of a drape 25, other separating means 3 such as wires or cup-shaped segments can be used as well.

Fig. 9 shows a sixth embodiment of the ocular speculum according to the invention. This embodiment comprises a substantially U- or V-shaped spring lever 30 having legs 31A and 31B that are provided with hooks 32A and 32B near each end. The hooks 32A and 32B form the separating means and each carry a substantially arcuate segment 21A and 21B respectively, forming the anchoring means to be inserted into and to be retained in the conjunctival fornixof an eye as discussed above. The arcuate segments are in this embodiment thus attached to the spring lever via an intermediate structure that forms the separating means. In working position, the spring lever thus extends over the outer portion of the eyelids and over the face. Advantageously, a surgical drape 25 is attached to the anchoring means and extends over the spring lever to prevent obstruction of the surgical field.

It will be clear that the invention is not limited to the described preferred embodiments and that many variations are possible. For example, the anchoring means may comprise several ring segments and may be substantially resilient or flexible, as long as sufficient anchoring or retaining capacity in the conjunctival fornix is provided, e.g. by biassing flexible arcuate segments to form a substantially annular structure for cooperation with the conjunctival fornix. The anchoring means may also comprise a tube-like hollow, flexible ring, wherein stiffening means are inserted, e.g. a stiffening wire, after the tube-like element is placed within the conjunctival fornix. Furthermore, the anchoring means and/or the spring lever may be curved along a substantially convex plane to follow the curvature of the head, such that insertion is facilitated and the risk of obstruction of the surgical field by the spring lever is reduced. Also, other advantageous embodiments can be obtained by combining features of the described preferred embodiments.

Such embodiments are within the scope of the following claims.

## Claims

1. Ocular speculum, comprising separating means for separating the eyelids of an eye by engagement of the ocular side of the eyelids, wherein said separating means comprise anchoring means for, in use, retaining said separating means within the conjunctival fornix of an eye.

2. Ocular speculum according to claim 1, wherein said anchoring means comprise a substantially annular element.

3. Ocular speculum according to claim 1, wherein said anchoring means comprise at least one substantially arcuate segment.

4. Ocular speculum according to any of the preceding claims, wherein said anchoring means comprise resilient elements that are biassed to form a substantially rigid, annular or arcuate structure.

5. Ocular speculum according to claim 3 or 4, wherein said anchoring means comprise two substantially arcuate segments connected by a spring lever.

6. Ocular speculum according to any of the preceding claims, wherein said separating means comprise a surgical drape extending from said anchoring means, such that an open area of said drape is at least partially surrounded by said anchoring means to allow passage of a portion of an eyeball.

7. Ocular speculum according to claim 6, wherein said surgical drape is at least partially transparent.

8. Ocular speculum according to claims 6 or 7, wherein said surgical drape comprises adhesive areas.

9. Ocular speculum according to at least claim 5 and claim 6, wherein a facial side of the surgical drape is arranged to extend over the spring lever.

10. Ocular speculum according to any of the preceding claims, wherein said separating means comprise a number of wires extending from the anchoring means.

11. Ocular speculum according to any of the preceding claims wherein said anchoring means are integrally formed with said separating means.

12. Ocular speculum according to any of the preceding claims, wherein said separating means comprise curved blades for cooperation with the ocular side of the eyelid, which blades extend from a lower portion forming said anchoring means.

13. Ocular speculum according to claim 12 wherein said anchoring means comprise two arcuate segments being resiliently connected by a spring lever via an intermediate structure that forms the separating means.

14. Ocular speculum according to any of the preceding claims, wherein said anchoring means are curved along a substantially convex plane.

15. A method for separation of the eyelids of an eye, wherein anchoring means of an ocular speculum are positioned in the conjunctival fornix of the eye to retain separating means of the speculum within the conjunctival fornix of the eye and wherein subsequently the eyelids of the eye are separated by moving a portion of the retained separating means outward relatively to the eye to engage the ocular side of the eyelids.

16. A method according to claim 15, wherein said separating means are subsequently attached to the face of a patient.

17. Surgical drape for use with an ocular speculum according to claims 1-14.
